(19) **Europäisches Patentamt**

**European Patent Office**

**Office européen des brevets**

(11) **EP 0 894 765 B1**

(12) **FASCICULE DE BREVET EUROPEEN**

(45) Date de publication et mention
de la délivrance du brevet:
**07.03.2001 Bulletin 2001/10**

(51) Int Cl.⁷: **C01B 7/07**

(21) Numéro de dépôt: **98401632.9**

(22) Date de dépôt: **01.07.1998**

(54) **Purification de l'acide chlorhydrique sous-produit de la synthèse de l'acide méthanesulfonique**

Reinigung von Salzsäure, Nebenprodukt der Methansulfonsäureherstellung

Purification of hydrochloric acid, by-product of the methanesulphonic acid synthesis

(84) Etats contractants désignés:
**AT BE CH DE DK ES FI FR GB IE IT LI LU NL SE**

(30) Priorité: **31.07.1997 FR 9709779**

(43) Date de publication de la demande:
**03.02.1999 Bulletin 1999/05**

(73) Titulaire: **Atofina**
**92800 Puteaux (FR)**

(72) Inventeurs:
• **Ollivier, Jean**
**64260 Arudy (FR)**
• **Clair, René**
**Saint-Pierre, 13500 Martigues (FR)**
• **Molines, Denise**
**69230 Saint-Genis Laval (FR)**
• **Ferrigno, Marc**
**64140 Billere (FR)**

(74) Mandataire: **Leboulenger, Jean**
**Atofina**
**D.C.R.D./D.P.I.**
**4, Cours Michelet**
**La Défense 10**
**92091 Paris la Défense Cedex (FR)**

(56) Documents cités:
EP-A- 0 675 107          GB-A- 740 252
GB-A- 1 350 328          US-A- 4 280 966

## Description

La présente invention concerne le domaine de l'acide méthanesulfonique (ci-après AMS) et a plus particulièrement pour objet un procédé de purification de l'acide chlorhydrique sous-produit lors de la synthèse de l'AMS.

La synthèse d'AMS à partir de méthylmercaptan et de chlore selon la réaction :

$$CH_3SH + 3\ Cl_2 + 3\ H_2O \rightarrow CH_3SO_3H + 6\ HCl$$

sous-produit une grande quantité d'acide chlorhydrique. Le flux gazeux d'HCl se sépare du milieu réactionnel en entraînant une partie des composés volatils et cet entraînement est d'autant plus important que la réaction s'opère à 100°C. Par des moyens connus en soi, on élimine facilement le chlore et le méthylmercaptan de l'acide chlorhydrique. L'eau n'est pas gênante dans la mesure où l'objectif est de récupérer une solution à 33 % d'HCl dans l'eau.

Cependant, au cours de la réaction qui conduit à l'acide méthanesulfonique, on produit un intermédiaire relativement volatil, le chlorure de méthanesulfonyle $CH_3SO_2Cl$(CMS) qui, lorsqu'on effectue l'abattage de l'HCl gazeux par l'eau, se transforme en acide méthanesulfonique, polluant par là-même la solution chlorhydrique et la rendant impropre à un certain nombre d'applications.

L'entraînement de CMS est important car le contact entre l'HCl et le CMS se fait à température élevée. L'excès de CMS est facilement enlevé en utilisant les techniques classiques connues telle que, par exemple, le lavage de l'effluent chlorhydrique par l'AMS produit dans la réaction et préférablement purifié. Néanmoins, des quantités non négligeables de CMS sont entraînées par l'HCl car les opérations ne se passent pas à basse température, mais au voisinage de la température ambiante. On sait qu'en fonction de la température (voir Tableau I), la charge en CMS de l'acide chlorhydrique prend des valeurs croissantes.

TABLEAU I

| Température (°C) | CMS dans HCl gaz (ppm) | AMS dans HCl aqueux 33 % (ppm) |
|---|---|---|
| -5 | 728 | 203 |
| 0 | 1245 | 350 |
| 5 | 1770 | 495 |
| 8 | 2570 | 720 |
| 12 | 4610 | 1290 |
| 15 | 5440 | 1520 |
| 18 | 7120 | 1990 |
| 21 | 8830 | 2470 |

Dans un procédé habituel de synthèse de l'AMS, le flux d'HCl est à une température voisine de 20°C. Il contient donc de 5000 à 9000 ppm de CMS qui conduisent à une solution chlorhydrique 33% contenant de 1500 à 2500 ppm d'AMS.

Le but de la présente invention est de fournir un procédé permettant de réduire le plus possible la teneur en CMS dans l'HCl gazeux, sans nécessiter la mise en oeuvre de températures extrêmement basses ou le recours à des technologies très sophistiquées et coûteuses.

Selon l'invention, on parvient à ce résultat par un procédé qui consiste essentiellement à amener la température du flux gazeux d'HCl à une température inférieure ou égale à 15°C et à injecter dans le flux une quantité d'eau allant de 0,01 à 20 % par rapport à la masse d'HCl à traiter.

Les opérations de refroidissement et d'injection d'eau peuvent être effectuées simultanément ou successivement. Dans ce dernier cas, on préfère d'abord injecter l'eau et refroidir ensuite.

Conformément au procédé selon l'invention, l'abaissement de la température de l'HCl gazeux jusqu'à une valeur inférieure ou égale à 15°C, de préférence entre environ -5 et +5°C, permet de diminuer considérablement la teneur en CMS. Ainsi, par exemple, le gaz refroidi de 21°C à 0°C passe d'une teneur de 8830 ppm de CMS à une teneur de 1245 ppm, soit une efficacité de 86 % ou un facteur de réduction égal à 7.

Conformément à la seconde caractéristique du procédé selon l'invention, on injecte dans le gaz chlorhydrique un aérosol d'eau pure en une quantité correspondant à quelques pour-cent de la masse d'HCl à traiter (0,01 à 20 %, de préférence 5 à 10 %). L'effet d'agrégation entre l'acide chlorhydrique et l'eau qui en résulte permet de piéger la majeure partie du CMS résiduel, que l'on peut renvoyer au réacteur de synthèse de l'AMS. Ainsi, avec 10 % d'eau par rapport à l'HCl, on passe facilement d'une teneur en CMS de 1245 ppm à 120 ppm, soit pour le procédé une efficacité globale d'épuration de 98,6 % ou un facteur de réduction égal à 73,6. Le procédé selon l'invention permet non seulement de purifier l'HCl, mais aussi de récupérer plus de 17 tonnes de CMS pour 1000 tonnes d'AMS produites.

L'exemple suivant illustre l'invention sans la limiter.

### EXEMPLE

#### *Appareillage*

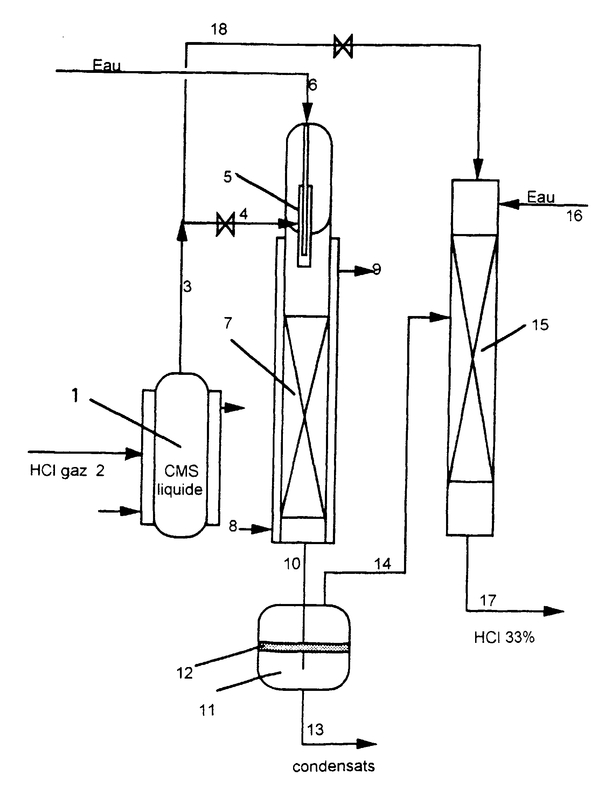

On a utilisé le dispositif décrit dans la figure unique annexée. Ce dispositif conçu pour traiter 60 litres/heure de gaz chlorhydrique (environ 90 g/h) est en verre avec des canalisations de liaison en PTFE. On a utilisé du CMS pur comme fluide de saturation de l'HCl.

A travers le saturateur (1) contenant du CMS liquide, on fait passer un courant (2) d'HCl gazeux et le courant sortant d'HCl chargé en CMS est amené via les conduits (3) et (4) dans une colonne de traitement comportant trois parties principales, à savoir :

- un injecteur à gaz (5) comportant un système de pulvérisation d'eau (mini hydro-injecteur) alimenté en (6) par une pompe à seringue ou par une micro pompe péristaltique,
- une zone d'échange thermique (7) constituée par un serpentin parcouru (8 et 9) par un liquide réfrigérant, et
- une zone de rétention (11) séparée du reste de la colonne par un dévésiculeur (12) en laine de roche tassée de 5 centimètres d'épaisseur et préalablement lavée à l'acide chlorhydrique concentré, rincée à l'eau distillée et séchée à l'éther avant son installation.

Après passage au travers de la zone d'échange thermique (7), l'acide chlorhydrique et les condensats sont amenés par la conduite (10) dans la zone de rétention (11). Les condensats sortent par la conduite (13). L'acide chlorhydrique traverse le dévésiculeur (12) et est amené par la conduite (14) dans une colonne (15) d'abattage par l'eau du gaz HCl épuré. Cette colonne (15) classique, alimentée en eau par la conduite (16), est thermostatée et fonctionne en batch pour produire en (17) de l'acide chlorhydrique titrant environ 33 %.

La même colonne a été utilisée pour contrôler le titre CMS de l'HCl pollué à traiter, en faisant circuler le flux gazeux d'HCl chargé en CMS directement du saturateur (1) à la colonne (15) via les conduits (3) et (18).

#### *Procédure*

On a diffusé de l'acide chlorhydrique gazeux anhydre dans du CMS liquide dont la température était fixée de façon à charger l'HCl avec la quantité désirée de CMS vapeur. Après passage dans la colonne de traitement, le gaz chlorhydrique était abattu à l'eau de façon à obtenir une solution à 33 % massique. Le CMS entraîné par l'HCl était hydrolysé en AMS que l'on a dosé selon les techniques analytiques connues.

Les résultats obtenus sont consignés dans le tableau suivant.

TABLEAU II

| Essais | ppm CMS dans HCl gaz | Température de condensation (°C) | Eau ajoutée à HCl (%) | ppm CMS dans HCl gaz traité | Rendement de l'opération (%) |
|---|---|---|---|---|---|
| 1 | 1775 | 0 | - | 1245 | 30 |
| 2 | 1775 | 0 | 12 | 24 | 98,6 |
| 3 | 2250 | 0 | - | 1245 | 44,6 |
| 4 | 2250 | 0 | 6 | 25 | 98,9 |
| 5 | 4610 | 0 | - | 1245 | 72 |
| 6 | 4610 | 0 | 10 | 270 | 94 |
| 7 | 5440 | 0 | - | 1245 | 77 |
| 8 | 5440 | 0 | 10 | 140 | 97,4 |
| 9 | 7120 | 5 | - | 1770 | 75 |
| 10 | 7120 | 5 | 10 | 250 | 96,4 |
| 11 | 8830 | -5 | - | 730 | 91 |
| 12 | 8830 | 0 | 10 | 80 | 99 |

**Revendications**

1. Procédé de purification de l'acide chlorhydrique sous-produit de la synthèse de l'acide méthanesulfonique caractérisé en ce que, pour éliminer le chlorure de méthanesulfonyle entraîné par l'HCl gazeux, on injecte dans le flux gazeux d'HCl une quantité d'eau allant de 0,01 à 20 % par rapport à la masse d'HCl à traiter et on abaisse la température à une température inférieure ou égale à 15°C.

2. Procédé selon la revendication 1, dans lequel on abaisse la température du flux gazeux d'HCl à une valeur comprise entre environ -5 et +5°C.

3. Procédé selon la revendication 1 ou 2, dans lequel on injecte 5 à 10 % d'eau par rapport à la masse d'HCl à traiter.

**Patentansprüche**

1. Verfahren zur Reinigung von Salzsäure, die ein Nebenprodukt bei der Herstellung von Methansulfonsäure ist, dadurch gekennzeichnet, daß man zur Entfernung des von dem gasförmigen HCl mitgeführten Methansulfonylchlorid in den gasförmigen HCl-Strom eine Wassermenge von 0,01 bis 20 %, bezogen auf die Menge des zu behandelnden HCl, einspritzt und die Temperatur auf eine Temperatur von 15 °C oder weniger absenkt.

2. Verfahren nach Anspruch 1, in dem man die Temperatur des gasförmigen HCl-Stroms auf einen Wert zwischen ungefähr -5 und +5 °C absenkt.

3. Verfahren nach Anspruch 1 oder 2, in dem man 5 bis 10 % Wasser, bezogen auf die Menge des zu behandelnden HCl, einspritzt.

**Claims**

1. Process for the purification of the hydrochloric acid by-product of the synthesis of methanesulphonic acid, characterized in that, in order to remove the methanesulphonyl chloride entrained by the HCl gas, an amount of water ranging from 0.01 to 20%, relative to the mass of HCl to be treated, is injected into the HCl gas flow and the temperature is lowered to a value below or equal to 15°C.

2. Process according to Claim 1, in which the temperature of the HCl gas flow is lowered to a value of approximately between -5 and +5°C.

3. Process according to Claim 1 or 2, in which 5 to 10% of water, relative to the mass of HC1 to be treated, is injected.